# EUROPEAN PATENT SPECIFICATION

(11) **EP 4 447 909 B1**
(45) Date of publication and mention of the grant of the patent: **04.11.2026**
(21) Application number: 22826089.9
(22) Date of filing: 01.12.2022
(51) Int. Cl.: A61K 8/25, A61K 8/895, A61K 8/37, A61Q 19/08, A61K 8/92, A61Q 19/00

(54) **A SKIN CARE COMPOSITION**
HAUTPFLEGEZUSAMMENSETZUNG
COMPOSITION DE SOIN DE LA PEAU

(30) Priority: 15.12.2021 WO PCT/CN2021/138344; 13.01.2022 EP 22151299
(43) Date of publication of application: 23.10.2024
(73) Proprietor: Unilever IP Holdings B.V., 6708 WH Wageningen (NL); Unilever Global IP Limited, Wirral, Merseyside CH62 4ZD (GB)
(72) Inventor: LOU, Anjing, 6708 WH Wageningen (NL); WANG, Lin, 6708 WH Wageningen (NL); YI, Shangchun, 6708 WH Wageningen (NL)
(74) Representative: Unilever Patent Group
(86) International application number: PCT/EP2022/083959
(87) International publication number: WO 2023/110410

(56) References cited:
- WO-A1-2017/220310
- CA-C- 2 785 753
- KR-B1- 102 309 728
- US-A1- 2018 153 792
- US-A1- 2021 275 409
- US-B2- 8 747 927

## Description

### Field of the Invention

The present invention relates to a skin care composition. In particular, the present invention relates to a skin care composition which has no problem of pilling when applied onto the skin surface.

### Background of the Invention

Ageing brings with it many changes to the appearance of skin. Of particular concern to individuals wishing to maintain a youthful appearance is the reduction or elimination of skin imperfections such as wrinkles, age spots or general unevenness of skin tone.

Thus, there has been considerable effort by the cosmetics industry to provide compositions which can mask or at least attenuate skin imperfections. One of the most important approaches is referred to as achieving blurring effect. Here the incoming light is distorted by scattering (lensing). Components of the cosmetic composition in this mechanism operate as lenses to bend and twist light in a variety of directions.

To achieve the blurring effect, particles may be employed. International application with publication number WO 2017/220310 A1 (Unilever) discloses composition containing porous silica and silicone elastomer and retinoid with a boosted blurring efficacy.

However, unfortunately, the present inventors have surprisingly found that the inclusion of high dosage of particles such as silicone elastomer, may cause problem to the composition. For example, when the skin care compositions containing high dosage of silicone elastomer were applied onto skin surface, tiny flakes or globs may be firmed on the skin surface, usually called pilling problem, leading to unfavorable experience to the consumers.

Therefore, the present inventors have recognised that there remains a need to improve the compositions containing silicone elastomer and porous silica. It was unexpectedly found that by including triglyceride with certain amount into the composition containing porous silica and silicone elastomer, the pilling problem was solved.

### Summary of the Invention

In a first aspect, the present invention provides a skin care composition comprising: (a) porous silica; (b) at least 8% of silicone elastomer by weight of the composition; (c) triglyceride; (d) at least 20% of water by weight of the composition, wherein the weight ratio of the triglyceride to the silicone elastomer is no less than 1:12. and the porous silica has an average diameter of 200 nm to 40 microns, and wherein "porous silica" refers to a silica particle with distributed voids throughout the entire volume of the particle; and wherein "diameter" refers to particle diameter in non-aggregated state, and wherein "average diameter" means the z-average diameter of the particles as measured using dynamic light scattering according to international standard ISO 13321, or the apparent volume median diameter (D50) of the particles as measured using laser diffraction according to international standard ISO 13320.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

"Silicone elastomer" as used herein refers to deformable organopolysiloxane with viscoelastic properties.

"Specific surface area" as used herein refers to specific surface area determined according to Brunauer-Emmett-Teller method. The value of the specific surface area may be measured by following ASTM standard D 3663-78.

"Diameter" as used herein refers to particle diameter in non-aggregated state unless otherwise stated. For polydisperse samples having particulate with diameter no greater than 1 µm, diameter means the z-average diameter measured, for example, using dynamic light scattering (see international standard ISO 13321) with an instrument such as a Zetasizer Nano^{™} (Malvern Instruments Ltd, UK) unless otherwise stated. For polydisperse samples having particulate with diameter no less than 1 µm, diameter means the apparent volume median diameter (D50, also known as x50 or sometimes d(0.5)) of the particles measurable for example, by laser diffraction using a system (such as a Mastersizer^{™} 2000 available from Malvern Instruments Ltd) meeting the requirements set out in ISO 13320 unless otherwise stated.

"Porous silica" as used herein refers to a silica particle with distributed voids throughout the entire volume of the particle. The voids can be individual or connected by small size openings, similar to pore openings that separate larger spaces. Preferably, the porous silica is non-fumed silica. The porous silica is preferably hydrophilic and is even more preferably unmodified porous silica microsphere. Hydrophilic porous silica as used herein refers to silica having a water absorption value of greater than 10g of water/100g of particle measured in same manner as described in ASTM Method D281-84 but using water instead of oil. Microsphere refers to spherical particle having average diameter of 0.1 to 50 microns, more preferably from 1 to 30 microns.

The specific surface area of the porous silica is preferably higher than 100 m²/g, more preferably from 200 to 1000 m²/g, even more preferably from 200 to 400 m²/g and most preferably from 200 to 300 m²/g.

The porous silica is capable for absorbing large amounts of oils. Preferably, the porous silica is a porous silica microsphere having an oil absorption value of higher than 50g/100g, more preferably higher than 100g/100g and even more preferably 120g/100g to 500g/100g. The oil absorption value refers to the values measured in conformity with ASTM Method D281-84.

The porous silica has an average diameter of 200 nm to 40 microns, more preferably from 0.6 to 25 microns, even more preferably from 1 to 20 microns, and most preferably from 2 to 18 microns. To have a better sensory, the porous silica is preferably substantially uniform in size which means less than 5% of the porous silica have a diameter less than 0.5 times the average diameter and less than 5% of the porous silica have a diameter greater than 1.5 times the average diameter. In another aspect, the range of the diameter of the porous silica is preferably 0.8 to 1.2 times the average diameter, more preferably 0.9 to 1.1 times the average diameter.

The porous silica is preferably present in amount of 0.01 to 20% by weight of the composition, more preferably from 0.05 to 14%, even more preferably from 0.2 to 9%, and most preferably from 0.5 to 5% by weight of the composition.

The composition comprises a silicone elastomer. It is highly preferred that the silicone elastomer is cross-linked. The silicone elastomer can be obtained from curable organo-polysiloxanes. Examples in this respect are: addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl terminated diorganopolysiloxane and SiH-containing diorganopolysiloxane; condensation-curing organopolysiloxane compositions which cure in the presence of an organotin compound or a titanate ester, by a condensation reaction between a hydroxyl terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation or electron beams. The silicone elastomer is preferably obtained by addition reaction-curing organopolysiloxane compositions which cure under platinum metal catalysis by the addition reaction between SiH-containing diorganopolysiloxane and organopolysiloxane having silicon-bonded vinyl groups

The silicone elastomer may either be an emulsifying or non-emulsifying cross-linked silicone elastomer or a combination thereof but preferably the silicone elastomer is non-emulsifying. The term "non-emulsifying," as used herein, defines cross-linked silicone elastomer from which poly-oxyalkylene units are absent. The term "emulsifying," as used herein, means cross-linked organopolysiloxane elastomer having at least one poly-oxyalkylene (e.g., poly-oxyethylene or polyoxypropylene) unit.

Preferred silicone elastomers are selected from organo-polysiloxanes available under the INCI names of dimethicone/vinyl dimethicone crosspolymer, dimethicone crosspolymer and Polysilicone-11. More preferably the silicone elastomer is dimethicone/vinyl dimethicone crosspolymer.

Typically the average diameter of the silicone elastomer is from 0.2 to 50 microns, more preferably from 0.5 to 20 microns, even more preferably from 0.8 to 10 microns, and still even more preferably from 1.5 to 6 microns.

The silicone elastomer is preferably present in amount of 8 to 50%, more preferably 10 to 42%, even more preferably 13 to 38%, and most preferably from 17 to 30% by weight of the composition.

To have a better blurring effect, opacity and/or viscosity stability, the weight ratio of silicone elastomer to the porous silica is preferably from 2:1 to 40:1, more preferably from 4:1 to 25:1 and even more preferably from 8:1 to 18:1.

The composition comprises triglyceride. Triglycerides are esters formed from glycerin and fatty acid. Preferably, the triglyceride is liquid at 25 °C and atmospheric pressure. Preferably, the triglyceride comprises caprylic/capric triglyceride, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil. More preferably the triglyceride comprises caprylic/capric triglyceride, coconut oil, jojoba oil, palm kernel oil, sunflower seed oil, safflower seed oil, cotton seed oil, olive oil or a mixture thereof. Even more preferably the triglyceride is selected from caprylic/capric triglyceride, coconut oil, jojoba oil, palm kernel oil, olive oil or a mixture thereof. Still even more preferably the triglyceride comprises caprylic/capric triglyceride and most preferably the triglyceride is caprylic/capric triglyceride.

The amount of triglyceride is 2 to 25%, more preferably 2 to 18%, even more preferably 2 to 15%, still even more preferably 4 to 15%, and most preferably 5 to 15% by weight of the composition.

To have a better blurring effect, opacity and/or less pilling issue, the weight ratio of the triglyceride to the silicone elastomer is preferably no less than 1:10, more preferably no less than 1:8 and even more preferably no less than 1:4. Preferably the weight ratio of the triglyceride to the silicone elastomer is preferably no greater than 5:1, more preferably no greater than 2:1 and even more preferably no greater than 1:1. Preferably, the weight ratio of the triglyceride to the porous silica is 1:2 to 25:1, more preferably 1:1 to 15:1, and even more preferably 2:1 to 10:1, and still even more preferably 3:1 to 8:1. Preferably, the weight ratio of the total weight of porous silica and silicone elastomer to the triglyceride is 1:5 to 13:1, more preferably 1:3 to 9:1 and still more preferably 1:1 to 6:1.

Preferably, the amount of triglyceride accounts for at least 20%, more preferably at least 40%, even more preferably at least 60% by weight based on the total weight of the triglyceride and cyclopentasiloxane in the composition. Preferably, the composition is essentially free of cyclopentasiloxane. "Essentially free of" as used herein means that the amount of cyclopentasiloxane is less than 0.5% by weight of the composition, preferably less than 0.1%, even more preferably less than 0.01% by weight of the composition.

The composition preferably comprises water in amount of 25 to 88% by weight of the composition, preferably from 30 to 88%, even more preferably from 38 to 78% by weight of the composition. The composition is preferably an emulsion and more preferably an oil-in-water emulsion.

The composition may comprise resorcinol derivative. Resorcinol derivative preferably refers to that at least one hydrogen on the ring structure and/or on a hydroxy group of the resorcinol replaced with an alkyl group, phenyl alkyl group. Preferably, the resorcinol derivative is 4-substituted resorcinol. Preferably, the resorcinol derivative is selected from 4-ethyl resorcinol, 4-butyl resorcinol, 4-hexyl resorcinol, phenylethyl resorcinol, or a mixture thereof, and more preferably, the resorcinol derivative comprises 4-hexyl resorcinol. The amount of the resorcinol derivative is preferably in the range of 0.00001 to 10%, more preferably from 0.001 to 5% and most preferably from 0.1 to 0.6% by weight of the total amount of the composition.

Preferably, the composition comprises Vitamin B3 compounds (including derivatives of vitamin B3). The vitamin B3 compounds comprises niacin, nicotinic acid, niacinamide or a mixture thereof. The most preferred vitamin B3 compound is niacinamide. Amount of Vitamin B3 compounds may be 0.1 to 10%, preferably 0.5 to 5% by weight of the composition.

Preferably, the composition comprises polyhydric alcohol. Polyhydric alcohols may be selected from group of glycerin, propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. Most preferred polyhydric alcohol is glycerol known also as glycerin. The amount of polyhydric alcohol may range anywhere from 0.1 to 20%, preferably 0.5 to 15% and more preferably 2 to 10% by weight of the composition.

Water-insoluble skin benefit agents may also be formulated into the compositions as conditioners and moisturizers. Examples include silicone oils; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid.

A skin care composition as used herein includes a composition for topical application to the skin of mammals, especially humans. Such a composition could be of the leave-on or of the wash-off type. Wash-off type is sometimes also referred to as rinse-off type. A leave-on composition means a composition that is applied to the desired skin surface and left on for example from one minute to 24 hours, after which it may be wiped or rinsed off with water, usually during the regular course of personal washing. A wash-off composition means a composition that is applied to the desired skin surface for a shorter period, for example, a few seconds or minutes and usually contains sufficient surfactants that aids in cleaning the surface which may be rinsed off with copious amounts of water. Preferably, the skin care composition is of the leave-on type. The composition of the present invention is in the form of a lotion or a cream.

The term "skin" as used herein includes the skin on the face (except eye lids and lips), neck, chest, abdomen, back, arms, under arms, hands, and legs. Preferably "skin" means includes the skin on the face (except eye lids and lips) and under arms, more preferably skin means skin on the face other than lips and eyelids.

The following examples are provided to facilitate an understanding of the invention. The examples are not intended to limit the scope of the claims.

### Examples

### Material

| Trade name | INCI name | Supplier | Diameter (microns) | Surface area (m²/g) | Oil Absorption (g/100g) |
|---|---|---|---|---|---|
| VALVANCE Touch 210 | Silica (Porous) | DSM | 6 to 15 | 232 | 150 |
| DC9509 (63% of solid active) | Dimethicone/Vinyl dimethicone Crosspolymer (and) C12-14 Pareth-12 | DOW CORNING | 3 to 5 | - | - |
| Crodamol^{™} GTCC | Caprylic/capric triglyceride | CRODA | - | - | - |

### Example 1

This example demonstrated the preparation of skin care compositions.

**Table 1**

| Ingredient | Samples (wt%)* | | | |
|---|---|---|---|---|
| | 1 | 2 | A | B |
| Water | Bal. | Bal. | Bal. | Bal. |
| VALVANCE Touch 210 | 2.00 | 2.00 | 2.00 | 2.00 |
| DC 9509 | 37.30 | 37.30 | 37.30 | 37.30 |
| Caprylic/Capric Triglyceride | 10.95 | 4.66 | - | 1.500 |
| Cyclopentasiloxane | - | - | 10.95 | 9.45 |
| Sodium acryloyldimethyltaurate copolymer and isohexadecane and water and polysorbate 80 | 1.50 | 1.50 | 1.50 | 1.50 |
| POE (20) sorbitan monolaurate | 1.00 | 1.00 | 1.00 | 1.00 |
| Phenoxyethanol | 0.50 | 0.50 | 0.50 | 0.50 |

| | | | | |
|---|---|---|---|---|
| *The level in this table refers the level of left-listed ingredients, unless otherwise specified. | | | | |

A series of skin care compositions were formulated as shown in Table 1.

### Example 2

This example demonstrated that the composition of the present invention had enhanced anti-pilling effect.

Bio-skin plates (color #50, Beaulax Co., Ltd., Tokyo, Japan) made of polyurethane elastomer were used as substrate to mimic the human skin. 20 µg of one sample as prepared in Example 1 was applied to and spread by finger cot within the circle with area of 7 cm² for pilling test and the sample was allowed to dry naturally for 10 minutes. The bio-skin plate was then rubbed by another clean bio-skin plate for 5 cycles. After rub, such clean bio-skin plate was observed by taking images by using Digieye system (Verivide, UK). If there were noticed flakes or globs on the clean bio-skin plate, it would be considered the the sample has the pilling problem. The results for all samples were recorded in Table 2.

**Table 2**

| Sample | Weight ratio of triglyceride to silicone elastomer | Pilling issue |
|---|---|---|
| 1 | 1:2.15 | No |
| 2 | 1:5.04 | No |
| A | 0 | Yes |
| B | 1:15.7 | Yes |

From the results, it was surprisingly found when the weight ratio of the triglyceride to the silicone elastomer was within the present invention, the samples (samples 1 and 2) had no pilling problems. In contrast, when, the weight ratio of the triglyceride to the silicone elastomer was outside the range of the present invention, the samples still had the pilling issue.

## Claims

1. A skin care composition comprising:
(a) porous silica;
(b) at least 8% of silicone elastomer by weight of the composition;
(c) 2 to 25% of triglyceride by weight of the composition; and
(d) at least 20% of water by weight of the composition,
wherein the weight ratio of the triglyceride to the silicone elastomer is no less than 1:12, and the porous silica has an average diameter of 200 nm to 40 microns, and wherein "porous silica" refers to a silica particle with distributed voids throughout the entire volume of the particle; and wherein "diameter" refers to particle diameter in non-aggregated state, and wherein "average diameter" means the z-average diameter of the particles as measured using dynamic light scattering according to international standard ISO 13321, or the apparent volume median diameter (D50) of the particles as measured using laser diffraction according to international standard ISO 13320.

2. The composition according to claim 1 wherein the porous silica has a specific surface area higher than 100 m²/g, determined according to Brunauer-Emmett-Teller method.

3. The composition according to claim 1 or 2 wherein the porous silica has an average diameter of from 1 to 20 microns.

4. The composition according to any one of the preceding claims wherein the amount of the porous silica is 0.01 to 20%, preferably from 0.2 to 9% by weight of the composition.

5. The composition according to any one of the preceding claims wherein the silicone elastomer is dimethicone/vinyl dimethicone crosspolymer.

6. The composition according to any one of the preceding claims wherein the average diameter of the silicone elastomer is from 0.2 to 50 microns, preferably from 1.5 to 6 microns.

7. The composition according to any one of the preceding claims wherein the silicone elastomer is present in amount of 8 to 50%, preferably 13 to 38% by weight of the composition.

8. The composition according to any one of the preceding claims wherein the triglyceride comprises caprylic/capric triglyceride, coconut oil, jojoba oil, palm kernel oil, sunflower seed oil, safflower seed oil, cotton seed oil, olive oil or a mixture thereof, preferably the triglyceride is caprylic/capric triglyceride.

9. The composition according to any one of the preceding claims wherein the amount of triglyceride is 2 to 18%, preferably 5 to 15% by weight of the composition.

10. The composition according to any one of the preceding claims wherein the weight ratio of the triglyceride to the porous silica is 1:2 to 25:1, preferably 2:1 to 10:1.

11. The composition according to any one of the preceding claims wherein the weight ratio of the total weight of porous silica and silicone elastomer to the triglyceride is 1:3 to 9:1 and preferably 1:1 to 6:1.

12. The composition according to any one of the preceding claims wherein the amount of triglyceride accounts for at least 20%, preferably at least 40% by weight based on the total weight of the triglyceride and cyclopentasiloxane in the composition.

13. The composition according to any one of the preceding claims wherein the amount of cyclopentasiloxane in the composition is less than 0.5% by weight of the composition, preferably less than 0.1%, more preferably less than 0.01% by weight of the composition.

14. The composition according to any one of the preceding claims wherein the composition is an oil-in-water emulsion.

15. The composition according any one of the preceding claims wherein the composition comprises water in amount of 25 to 88% by weight of the composition.

## Patentansprüche

1. Hautpflegezusammensetzung, umfassend:
(a) poröses Siliciumdioxid;
(b) mindestens 8 Gew.-% der Zusammensetzung an Silikonelastomer;
(c) 2 bis 25 Gew.-% der Zusammensetzung an Triglycerid; und
(d) mindestens 20 Gew.-% der Zusammensetzung an Wasser,
wobei das Gewichtsverhältnis von Triglycerid zu Silikonelastomer nicht kleiner als 1:12 ist und das poröse Siliciumdioxid einen mittleren Durchmesser von 200 nm bis 40 Mikrometern aufweist, und wobei sich "poröses Siliciumdioxid" auf ein Siliciumdioxidpartikel bezieht, dessen Hohlräume über das gesamte Volumen des Partikels verteilt sind; und wobei sich "Durchmesser" auf den Partikeldurchmesser im nicht aggregierten Zustand bezieht und wobei "mittlerer Durchmesser" den Z-mittleren Durchmesser der Partikel, gemessen mittels dynamischer Lichtstreuung nach der internationalen Norm ISO 13321, oder den scheinbaren volumetrischen Mediandurchmesser (D50) der Partikel, gemessen mittels Laserbeugung gemäß der internationalen Norm ISO 13320, bedeutet.

2. Zusammensetzung nach Anspruch 1, wobei das poröse Siliziumdioxid eine spezifische Oberfläche von mehr als 100 m²/g aufweist, bestimmt gemäß der Brunauer-Emmet-Teller-Methode.

3. Zusammensetzung nach Anspruch 1 oder 2, wobei das poröse Siliciumdioxid einen mittleren Durchmesser von 1 bis 20 Mikrometern aufweist.

4. Zusammensetzung nach irgendeinem der vorhergehenden Ansprüche, wobei die Menge an porösem Siliciumdioxid 0,01 bis 20 Gew.-%, vorzugsweise 0,2 bis 9 Gew.-% der Zusammensetzung beträgt.

5. Zusammensetzung nach irgendeinem der vorhergehenden Ansprüche, wobei das Silikonelastomer ein Dimethicon/Vinyldimethicon-Crosspolymer ist.

6. Zusammensetzung nach irgendeinem der vorhergehenden Ansprüche, wobei der mittlere Durchmesser des Silikonelastomers zwischen 0,2 und 50 Mikrometern, vorzugsweise zwischen 1,5 und 6 Mikrometern liegt.

7. Zusammensetzung nach irgendeinem der vorhergehenden Ansprüche, wobei das Silikonelastomer in einer Menge von 8 bis 50 Gew.-%, vorzugsweise von 13 bis 38 Gew.-% der Zusammensetzung vorliegt.

8. Zusammensetzung nach irgendeinem der vorhergehenden Ansprüche, wobei das Triglycerid Caprylic-/Capric-Triglycerid, Kokosnussöl, Jojobaöl, Palmkernöl, Sonnenblumenkernöl, Distelöl, Baumwollsamenöl, Olivenöl oder eine Mischung davon umfasst, wobei das Triglycerid vorzugsweise Caprylic-/Capric-Triglycerid ist.

9. Zusammensetzung nach irgendeinem der vorhergehenden Ansprüche, wobei die Menge an Triglycerid 2 bis 18 Gew.-%, vorzugsweise 5 bis 15 Gew.-% der Zusammensetzung beträgt.

10. Zusammensetzung nach irgendeinem der vorhergehenden Ansprüche, wobei das Gewichtsverhältnis von Triglycerid zu porösem Siliciumdioxid 1:2 bis 25:1, vorzugsweise 2:1 bis 10:1 beträgt.

11. Zusammensetzung nach irgendeinem der vorhergehenden Ansprüche, wobei das Gewichtsverhältnis des Gesamtgewichts von porösem Siliciumdioxid und Silikonelastomer zum Triglycerid 1:3 bis 9:1 und vorzugsweise 1:1 bis 6:1 beträgt.

12. Zusammensetzung nach irgendeinem der vorhergehenden Ansprüche, wobei die Menge an Triglycerid mindestens 20 Gew.-%, vorzugsweise mindestens 40 Gew.-%, bezogen auf das Gesamtgewicht des Triglycerids und des Cyclopentasiloxans in der Zusammensetzung, beträgt.

13. Zusammensetzung nach irgendeinem der vorhergehenden Ansprüche, wobei die Menge an Cyclopentasiloxan in der Zusammensetzung weniger als 0,5 Gew.-% der Zusammensetzung, vorzugsweise weniger als 0,1 Gew.-%, bevorzugter weniger als 0,01 Gew.-% der Zusammensetzung beträgt.

14. Zusammensetzung nach irgendeinem der vorhergehenden Ansprüche, wobei die Zusammensetzung eine Öl-in-Wasser-Emulsion ist.

15. Zusammensetzung nach irgendeinem der vorhergehenden Ansprüche, wobei die Zusammensetzung Wasser in einer Menge von 25 bis 88 Gew.-% der Zusammensetzung enthält.

## Revendications

1. Composition de soin de la peau comprenant :
(a) de la silice poreuse ;
(b) au moins 8 %, en poids de la composition, d'élastomère de silicone ;
(c) 2 à 25 %, en poids de la composition, de triglycéride ; et
(d) au moins 20 %, en poids de la composition, d'eau,
dans laquelle le rapport en poids du triglycéride à l'élastomère de silicone est d'au moins 1/12, et la silice poreuse a un diamètre moyen de 200 nm à 40 micromètres, et
dans laquelle "silice poreuse" se réfère à une particule de silice ayant des vides distribués dans tout le volume de la particule ; et dans laquelle "diamètre" se réfère au diamètre de particule dans un état non agrégé, et dans laquelle "diamètre moyen" signifie le diamètre moyen en z des particules tel que mesuré par diffusion dynamique de la lumière conformément à la norme internationale ISO 13321, ou le diamètre médian en volume apparent (D50) des particules tel que mesuré par diffraction laser conformément à la norme internationale ISO 13320.

2. Composition selon la revendication 1, dans laquelle la silice poreuse a une surface spécifique, déterminée conformément à la méthode de Brunauer-Emmett-Teller, supérieure à 100 m²/g.

3. Composition selon la revendication 1 ou 2, dans laquelle la silice poreuse a un diamètre moyen de 1 à 20 micromètres.

4. Composition selon l'une quelconque des revendications précédentes, dans laquelle la quantité de la silice poreuse est de 0,01 à 20 %, de préférence de 0,2 à 9 %, en poids de la composition.

5. Composition selon l'une quelconque des revendications précédentes, dans laquelle l'élastomère de silicone est un polymère réticulé de diméthicone/vinyldiméthicone.

6. Composition selon l'une quelconque des revendications précédentes, dans laquelle le diamètre moyen de l'élastomère de silicone est de 0,2 à 50 micromètres, de préférence de 1,5 à 6 micromètres.

7. Composition selon l'une quelconque des revendications précédentes, dans laquelle l'élastomère de silicone est présent en une quantité de 8 à 50 %, de préférence de 13 à 38 %, en poids de la composition.

8. Composition selon l'une quelconque des revendications précédentes, dans laquelle le triglycéride comprend du triglycéride caprylique/caprique, de l'huile de coco, de l'huile de jojoba, de l'huile de palmiste, de l'huile de tournesol, de l'huile de carthame, de l'huile de coton, de l'huile d'olive ou un mélange de ceux-ci, de préférence le triglycéride est un triglycéride caprylique/caprique.

9. Composition selon l'une quelconque des revendications précédentes, dans laquelle la quantité de triglycéride est de 2 à 18 %, de préférence de 5 à 15 %, en poids de la composition.

10. Composition selon l'une quelconque des revendications précédentes, dans laquelle le rapport en poids du triglycéride à la silice poreuse est de 1/2 à 25/1, de préférence de 2/1 à 10/1.

11. Composition selon l'une quelconque des revendications précédentes, dans laquelle le rapport en poids du poids total de la silice poreuse et de l'élastomère de silicone au triglycéride est de 1/3 à 9/1, et de préférence de 1/1 à 6/1.

12. Composition selon l'une quelconque des revendications précédentes, dans laquelle la quantité de triglycéride compte pour au moins 20 %, de préférence au moins 40 % en poids par rapport au poids total du triglycéride et du cyclopentasiloxane dans la composition.

13. Composition selon l'une quelconque des revendications précédentes, dans laquelle la quantité de cyclopentasiloxane dans la composition est inférieure à 0,5 % en poids de la composition, de préférence inférieure à 0,1 %, mieux encore inférieure à 0,01 % en poids de la composition.

14. Composition selon l'une quelconque des revendications précédentes, dans laquelle la composition est une émulsion huile dans l'eau.

15. Composition selon l'une quelconque des revendications précédentes, dans laquelle la composition comprend de l'eau en une quantité de 25 à 88 % en poids de la composition.
